# EUROPEAN PATENT APPLICATION

(11) **EP 2 578 202 A2**
(43) Date of publication of application: **10.04.2013**
(21) Application number: 11786922.2
(22) Date of filing: 26.05.2011
(51) Int. Cl.: A61K 6/04, A61K 6/02, C22C 5/00, C22C 38/00

(54) **METAL ALLOY FOR FUSION OF DENTAL CERAMICS, AND DENTAL PROSTHESIS**

(30) Priority: 24.11.2010 KR 20100117307; 27.05.2010 KR 20100049599
(71) Applicant: Park, Hyung-Seok, Seoul 152-100 (KR)
(72) Inventor: Park, Hyung-Seok, Seoul 152-100 (KR)
(74) Representative: Hall, Matthew Benjamin
(86) International application number: PCT/KR2011/003875
(87) International publication number: WO 2011/149290

(57) **Abstract**

Metal alloys for dental porcelain fusing and dental prosthetic appliances are provided. The metal alloy includes: gold (Au) in an amount that is greater than 0 and equal to or smaller than 35 weight%; silver (Ag) in an amount of 5 to 35 weight%; indium (In) in an amount of 6 to 40 weight%; palladium (Pd) in an amount of 30 to 70 weight%; and at least one element selected from the group consisting of boron (B), germanium (Ge), zirconium (Zr), silicon (Si) and iron (Fe) in a total amount that is greater than 0 and equal to or smaller than 5.0 weight%.

## Description

### TECHNICAL FIELD

The present invention relates to metal alloys, and in particular, to metal alloys for dental porcelain fusing that is used in prosthetic appliance in dental clinics and dental prosthetic appliances using the same.

### BACKGROUND ART

Metal alloys for dental porcelain fusing are used in mouths where various environmental changes of temperature, acidity, pressure, or the like in human bodies occur. Accordingly, they require not only excellent mechanical properties but also chemical stability and aesthetic appreciation. For example, since metal alloys for dental porcelain fusing are used in mouths, they should not corrode or discolor, and also should not be harmful for human bodies. In addition, since metal alloys for dental porcelain fusing endure a pressure caused by chewing food, they are required to have high mechanical properties.

From this point, gold-based alloys with high gold content are widely used for fusing with porcelain. However, gold-based alloys are expensive. Accordingly, alternative alloys that have similar properties to those of gold-based alloys and high economic properties are being developed. For example, a gold-palladium alloy, a silver-palladium alloy, a nickel-chromium alloy, or the like are being studied.

### DETAILED DESCRIPTION OF THE INVENTION

### TECHNICAL PROBLEM

However, it is not easy to develop alternative alloys with physical properties, chemical properties, and aesthetic appreciations that are suitable for fusing with porcelain. For example, a nickel-chromium alloy is cheap, but may cause allergic responses or accumulate heavy metal in vivo. Other alloys may cause formation of a discolored structure, may cause cytotoxicity, and may have a weak binding force with porcelain or poor molding properties. Thus, the present invention is introduced to resolve such problems. The described objectives are illustratively presented, and the present invention is not limited thereto.

### TECHNICAL SOLUTION

According to an aspect of the present invention, a dental metal alloy for dental porcelain fusing includes: gold (Au) in a content that is greater than 0 and equal to or smaller than 35 weight%; silver (Ag) in a content of 5 to 35 weight%; indium (In) in a content of 6 to 40 weight%; palladium (Pd) in a content of 30 to 70 weight%; and at least one element selected from the group consisting of boron (B), germanium (Ge), zirconium (Zr), silicon (Si) and iron (Fe) in a total content that is greater than 0 and equal to or smaller than 5.0 weight%.

According to an embodiment of the present invention, the metal alloy may further include at least one element selected from the group consisting of zinc (Zn), tin (Sn), gallium (Ga), selenium (Se), carbon (C), antimony (Sb), and copper (Cu) in a total content of greater than 0 and equal to or smaller than 10.0%.

According to another embodiment of the present invention, the metal alloy may further include at least one element selected from the group consisting of iridium (Ir), rhenium (Re), rhodium (Rh), ruthenium (Ru), lanthanum (La), osmium (Os), and bismuth (Bi) in a total content of greater than 0 and equal to or smaller than 2 weight%.

According to another embodiment of the present invention, the metal alloy may further include at least one element selected from the group consisting of chromium (Cr), niobium (Nb), magnesium (Mg), titanium (Ti), cobalt (Co), aluminum (Al), vanadium (V), tantalum (Ta), molybdenum (Mo) and tungsten (W) in a total content of greater than 0 and equal to or smaller than 10.0 weight%.

A dental metal alloy for dental porcelain fusing according to another embodiment of the present invention includes: gold (Au) in a content that is greater than 0 and equal to or smaller than 35 weight%; silver (Ag) in a content of 5 to 35 weight%; indium (In) in a content of 12.0 to 35.0 weight%; at least one element selected from the group consisting of zinc (Zn), tin (Sn), gallium (Ga), selenium (Se), carbon (C), antimony (Sb), and copper (Cu) in a total content of greater than 0 and equal to or smaller than 10.0 weight%; at least one element selected from the group consisting of boron(B), germanium (Ge), zirconium (Zr), silicon (Si) and iron (Fe) in a total content of greater than 0 and equal to or smaller than 10.0 weight%; at least one element selected from the group consisting of chromium (Cr), niobium (Nb), magnesium (Mg), titanium (Ti), cobalt (Co), aluminum (Al), vanadium (V), tantalum (Ta), molybdenum (Mo), and tungsten (W) in a total content of greater than 0 and equal to or smaller than 10.0 weight%; at least one element selected from the group consisting of iridium (Ir), rhenium (Re), rhodium (Rh), ruthenium (Ru), lanthanum (La), osmium (Os), and bismuth (Bi) in a total content of greater than 0 and equal to or smaller than 2 weight%; and residual palladium (Pd) and inevitable impurities.

According to an embodiment of the present invention, indium may have a content of 12.0 to 26.0 weight%, and a melting point of 1030 to 1200°C.

According to an embodiment of the present invention, indium (In) has a content of 20.3 to 30.0 weight%, and a melting point of 920 to 1040°C.

A metal alloy for dental porcelain fusing according to another embodiment of the present invention, includes: gold (Au) in a content that is greater than 0 and equal to or smaller than 85 weight%; silver (Ag) in a content that is greater than 0 and equal to or smaller than 35 weight%; indium (In) in a content of 6 to 40 weight%; palladium (Pd) in a content of 5 to 70 weight%; at least one first element selected from the group consisting of boron (B), germanium (Ge), zirconium (Zr), silicon (Si) and iron (Fe) in a total content that is greater than 0 and equal to or smaller than 5.0 weight%; and at least one second element selected from the group consisting of chromium (Cr), niobium (Nb), magnesium (Mg), titanium (Ti), cobalt (Co), aluminum (Al), vanadium (V), tantalum (Ta), molybdenum (Mo) and tungsten (W) in a total content of greater than 0 and equal to or smaller than 10.0 weight%.

According to an embodiment of the present invention, the at least one element may include silicon (Si) in a content that is greater than 0 and smaller than 1.0 weight%, and/or the at least one second element includes cobalt (Co) in a content of 0 to 5.0 weight%. Furthermore, a calcination temperature of the metal alloy and ceramic porcelain is in a range of 900 to 980 °C.

According to another embodiment of the present invention, the metal alloy may further include at least one third element selected from the group consisting of zinc (Zn), tin (Sn), gallium (Ga), selenium (Se), carbon (C), antimony (Sb), and copper (Cu) in a total content that is greater than 0 and equal to or smaller than 10.0 weight%.

According to another embodiment of the present invention, the at least one element may include silicon (Si) in a content that is greater than 0 and smaller than 1.0 weight%, the at least one second element may include cobalt (Co) in a content of 0 to 5.0 weight%, and at least one third element may include a copper (Cu) in a content that is greater than 0 and equal to or smaller than 5.0 weight%.

According to another aspect of the present invention, a dental prosthetic appliance may be manufactured by using at least one of the dental metal alloys described above.

According to another aspect of the present invention, a method of manufacturing a dental prosthetic appliance may include providing the dental metal alloy; pretreating the metal alloy to remove a metal oxide on a surface thereof; and fusing ceramic porcelain on the metal alloy at a calcination temperature range of 900 to 980°C.

### ADVANTAGEOUS EFFECTS

Metal alloys for dental porcelain fusing according to some embodiments of the present invention have high economic properties due to high palladium and indium contents, and also have excellent mechanical properties, chemical stability, and high in-vivo compatibility due to the inclusion of other additional elements

Metal alloys for dental porcelain fusing according to some embodiments of the present invention have an appropriately controlled alloy component ratio of a palladium-indium-gold-silver based alloy, and thus, when calcined at high temperature with ceramic porcelain, deformation of the alloy may be prevented.

### DESCRIPTION OF THE DRAWINGS

FIG. 1 is a picture of a dental prosthetic appliance that is prepared by clacination without a pretreatment according to Comparative Example.
FIG. 2 is a picture of a dental prosthetic appliance that is prepared by clacination with a pretreatment according to Experimental Example.

### BEST MODE

Hereinafter, the present invention is to be described in detail with reference to embodiments. However, the present invention is not limited to the following embodiments, and may be embodied in various other forms, and the present embodiments are provided to make the disclosure of the present invention complete and to fully inform one of ordinary skill in the art of the scope of the present invention.

In embodiments of the present invention, weight% (wt%) is a percentage point of the weight of a component based on a total weight of the entire alloy. Regarding ranges with respect to weight%, when the expressions of 'greater than or smaller than' are used, a boundary value of a set range is not included, and when only the expression of 'range' is used, or the expression of 'equal to or greater than, or equal to or smaller than' are used, the boundary value of the range is included.

In embodiments of the present invention, inevitable impurities may indicate typical impurities that may be introduced without any intension during metal alloys or prosthetic appliances are manufactured.

A dental metal alloy for dental porcelain fusing according to an embodiment of the present invention may be formed by mixing palladium (Pd) with gold (Au), silver (Ag), and a first adding element. Palladium is a major element and may be provided as an alternative to gold and platinum, and is known as being harmless to human bodies. A content of palladium may vary according to contents of gold, silver, and adding elements, and palladium may be selected together with gold, silver, and adding elements to exert appropriate performance of alloys for dental porcelain fusing. For example, for a decrease in costs, palladium may occupy the residual of an alloy and may be included in a range of 30 to 70 weight%. According to an embodiment of the present invention, if the gold content is high, a content of palladium may be 5% or more. The metal alloy for dental porcelain fusing may include inevitable impurities that are originated from impurities of the elements or introduced without any intension in preparing alloys.

Gold may be added to improve erosion resistance, discoloration resistance, and elongation, and the content thereof may vary according to other elements, for example, the palladium content, and is not particularly limited. However, if a competitive price is required, the gold content may be restricted to 40 weight%, in particular, 35 weight% or less. In particular, when an alternative element content is high, the gold content may be restricted to 25 weight% or lower. In addition, a part of the gold may be replaced with platinum (Pt). In this case, a total content of gold and platinum may be 35 weight% or lower. Furthermore, platinum is expensive and has a high melting point, the platinum content in an alloy for molding may be restricted to 20 weight% or lower. However, if a competitive price is not required, the palladium content may be reduced and the gold content may be increased to about 85%.

Silver (Ag) may be added together with palladium to prevent formation of discolored product, fractures, hydrogen embrittlement, or a decrease in molding properties (greater than 0%). Furthermore, silver may be added in a content of at least 5 weight% to prevent formation of discolored structure and to improve elongation, and in consideration of yellowing, the silver content may be restricted to 35 weight% or lower. Under strict conditions, the silver content may be restricted to a range of 13 to 31 weight% to maintain the color of the alloy.

Indium (In) may be added to increase a binding force with porcelain and retain aesthetic appreciation. For example, to secure a minimum binding force with porcelain, a minimum indium content may be 3 weight%. When the indium content is at least 6 weight% or more, the alloy may be light yellow and a greater indium content leads to stronger yellow. Meanwhile, the greater indium content, the alloy has a lower melting point and a higher thermal expansion coefficient. Accordingly, the indium content is restricted to 40% or lower, and may be appropriately determined according to the calcination temperature of the alloy. Furthermore, to prevent excessive increase in hardness and deformation during binding with porcelain, the indium content may be further restricted to 35 weight% or less.

For example, in embodiments of the present invention, the calcination temperature range may vary according to ceramic powder. Roughly, a low temperature calcinations indicates a calcination temperature of about 800°C or lower, and a high temperature calcination indicates a calcination temperature of about 900 to 980°C.

The first adding element may include at least one element selected from the group consisting of boron (B), germanium (Ge), zirconium (Zr), silicon (Si) and iron (Fe). The first adding element may offset a decrease in melting point and an increase in thermal expansion coefficient of the alloy due to the increased indium content. The first adding element content may be in a range of 0.1 weight% to 10.0 weight% in consideration of an excess increase in melting point of the alloy and an excess decrease in thermal expansion coefficient of the alloy, and under strict conditions, the first adding element content may be restricted to 6.0 weight% or lower to suppress formation of an oxide, such as borax, and the oxide-induced discoloration of the alloy.

A dental metal alloy for dental porcelain fusing according to another embodiment of the present invention may be formed by mixing palladium (Pd) with gold (Au), silver (Ag), the first adding element, and a second adding element. As described above, the metal alloy for dental porcelain fusing may include inevitable impurities that are originated from impurities of the elements or introduced without any intension in preparing alloys. In the present embodiment, palladium (Pd), gold (Au), silver (Ag) and the first adding element will not described in detail herein because their description have been already presented above.

According to another embodiment of the present invention, the metal alloy may further include at least one element selected from the group consisting of chromium (Cr), niobium (Nb), magnesium (Mg), titanium (Ti), cobalt (Co), aluminum (Al), vanadium (V), tantalum (Ta), molybdenum (Mo) and tungsten (W) in a total content of greater than 0 and equal to or smaller than 10.0 weight%. Like the first adding element, the second adding element may offset a decrease in melting point and an increase in thermal expansion coefficient of the alloy due to the increased indium content. Thus, the second adding element may allow the indium content to increase in the alloy, the alloy may be more distinctive yellow.

Furthermore, the second adding element may suppress an increase in deformation due to the increased indium content when the metal alloy is calcined with ceramic porcelain. The indium contained in metal alloy may flow down together with ceramic porcelain when high temperature calcination is performed thereon with ceramic porcelain. Thus, when the indium content in the metal alloy is high, the metal alloy may be inappropriate for high temperature calcination. However, since the second adding element is combined with indium so that deformation of indium is suppressed during high temperature calcination, the indium content in metal alloy for high temperature calcination may increase.

For example, the second adding element may be included in a content of 0.1 weight% or more to suppress deformation of indium. The second adding element may be more strongly required as the calcination temperature increases. However, in consideration of discoloration and a decrease in molding properties, the second adding element content may be restricted to 10.0 weight% or less. Furthermore, in consideration of an excess increase in melting point and an excess decrease in thermal expansion coefficient, the second adding element content may be restricted to 5.0 weight% or less.

A dental metal alloy for dental porcelain fusing according to another embodiment of the present invention may be formed by mixing palladium (Pd) with gold (Au), silver (Ag), the first adding element, and a third adding element. As described above, the metal alloy for dental porcelain fusing may include inevitable impurities that are originated from impurities of the elements or introduced without any intension in preparing alloys. In the present embodiment, palladium (Pd), gold (Au), silver (Ag) and the first adding element will not described in detail herein because their description have been already presented above.

The third adding element may include at least one element selected from the group consisting of zinc (Zn), tin (Sn), gallium (Ga), selenium (Se), carbon (C), antimony (Sb), and copper (Cu). The third adding element may be added to preserve effects of an element that inhibits the embodiment of yellow alloy due to indium, for example, the second adding element. Furthermore, the third adding element may prevent discoloration of the first adding element. However, when the third adding element content increases, molding properties of the alloy may decrease, cytotoxicity may increase, a melting point may decrease, and an erosion resistance may decrease. Accordingly, a content of the third adding element may be restricted to 10.0 weight% or lower, and in the case of copper, to 5.0 weight% or lower.

A dental metal alloy for dental porcelain fusing according to another embodiment of the present invention may be formed by mixing palladium (Pd) with gold (Au), silver (Ag), the first adding element, and a fourth adding element. As described above, the metal alloy for dental porcelain fusing may include inevitable impurities that are originated from impurities of the elements or introduced without any intension in preparing alloys. In the present embodiment, palladium (Pd), gold (Au), silver (Ag) and the first adding element will not described in detail herein because their description have been already presented above.

The fourth adding element may include at least one element selected from the group consisting of iridium (Ir), rhenium (Re), rhodium (Rh), ruthenium (Ru), lanthanum (La), osmium (Os), and bismuth (Bi) in a total content of greater than 0 and equal to or smaller than 2 weight%. The fourth adding element may improve molding properties, or may contribute grain refinement of the alloy, thereby increasing rigidity thereof. Accordingly, the fourth adding element content needs to be controlled in consideration of these properties. For example, when the fourth adding element content is greater than 2 weight%, the grain refinement effects may decrease. Accordingly, the fourth adding element content needs to be controlled to be 2 weight% or less.

A dental metal alloy for dental porcelain fusing according to another embodiment of the present invention may be formed by mixing palladium (Pd) with gold (Au), silver (Ag), the first adding element, and additionally, two or more of the second through fourth adding elements. As described above, the metal alloy for dental porcelain fusing may include inevitable impurities that are originated from impurities of the elements or introduced without any intension in preparing alloys. Functions of the respective elements may be understood by referring to corresponding descriptions in the embodiments above.

The metal alloys for dental porcelain fusing according to embodiments of the present include lower expensive gold and platinum contents and instead, higher palladium and indium contents, and thus, have high price competitiveness. In addition, due to the inclusion of other adding elements, excellent mechanical properties, chemical stability, and high in vivo compatibility may be obtained.

A dental prosthetic appliance according to an embodiment of the present invention may be prepared by using such metal alloys for dental porcelain fusing. For example, a dental prosthetic appliance may be formed by attaching ceramic porcelain onto such metal alloys. A dental prosthetic appliance formed as described above may have not only excellent mechanical and chemical properties of the metal alloy and but also aesthetic appreciation of the ceramic porcelain, and thus, have high marketability.

Hereafter, Comparative Example and Experimental Examples are provided to promote understanding of the present invention. However, Comparative Examples and Experimental Examples are presented for illustrative purpose only, and do not limit the scope of the present invention.

The inventors of the present application paid attention to a palladium-indium-based alloy as an alternative to expensive gold and platinum. First, they investigated physical properties according to a composition ratio of the palladium-indium-based alloy. Table 1 shows composition ratios and physical properties of the palladium-indium-based alloy prepared according to Experimental Example I. Samples shown in Table 1 were prepared as follows: alloy components were dissolved and then injected into a graphite mold to form a mold product, and the mold product was extended by using a roller and then imprinted and cut to produce samples.

**[Table 1]**

| Experimental Example I | Alloy element (weight%) | | | | Physical characteristics | | |
|---|---|---|---|---|---|---|---|
| | Pd | Au | Ag | In | Thermal expansion coefficient(10⁻⁶K⁻¹) | Melting point (°C) | Color |
| 1 | 50 | | | 50 | 18.41 | 803 | yellow |
| 2 | 55 | | | 45 | 17.75 | 867 | yellow |
| 3 | 60 | | | 40 | 17.20 | 932 | yellow |
| 4 | 62 | | | 38 | 16.82 | 958 | yellow |
| 5 | 64 | | | 36 | 16.44 | 984 | yellow |
| 6 | 66 | | | 34 | 16.06 | 1010 | yellow |
| 7 | 68 | | | 32 | 15.68 | 1036 | yellow |
| 8 | 70 | | | 30 | 15.36 | 1062 | yellow |
| 9 | 35 | 3 | 35 | 27 | 18.02 | 918 | yellow |
| 10 | 39 | 6 | 35 | 20 | 17.17 | 998 | yellow |
| 11 | 41 | 9 | 35 | 15 | 16.58 | 1051 | yellow |
| 12 | 41 | 12 | 35 | 12 | 16.26 | 1079 | yellow |
| 13 | 41 | 15 | 35 | 9 | 15.93 | 1107 | light yellow |
| 14 | 39 | 20 | 35 | 6 | 15.65 | 1127 | light yellow |

Referring to Table 1, the greater indium content, the lower melting point of the alloy. This is because the melting point of indium is much lower than those of gold and silver. When a calcination temperature is considered in preparing a dental prosthetic appliance, a melting point of the metal alloy for dental porcelain fusing needs to be 920°C or more, and accordingly, the indium content may be restricted to 40 weight% or less. Meanwhile, to maintain the color of the alloy for fusing with porcelain to be yellow, the indium content needs to be 6 weight% or more, and in particular to obtain more dark yellow, the indium content may be maintained to be 12 weight% or more. Considering these results, in following Experimental Examples, the indium was added in a content range of 12 to 40 weight%.

Table 2 shows the alloy composition (weight%) and physical properties of Experimental Examples according to the present invention.

**[Table 2]**

| Experimental Example II | Alloy element | | | | | | | | | Physical characteristics |
|---|---|---|---|---|---|---|---|---|---|---|
| | Au | Pd | Ag | Zn | In | Si | Ge | Zr | Co | Thermal expansion coefficient (10⁻⁶K⁻¹) |
| 1 | 10 | 45 | 17.5 | 0.5 | 27 | 0 | | | | 16.96 |
| 2 | 10 | 45 | 17 | 0.5 | 27 | 0.5 | | | | 14.90 |
| 3 | 10 | 45 | 16.5 | 0.5 | 27 | 1 | | | | 12.94 |
| 4 | 10 | 45 | 15.5 | 0.5 | 27 | 2 | | | | 8.80 |
| 5 | 10 | 45 | 17.5 | 0.5 | 27 | | 0 | | | 16.96 |
| 6 | 10 | 45 | 17 | 0.5 | 27 | | 0.5 | | | 16.04 |
| 7 | 10 | 45 | 16.5 | 0.5 | 27 | | 1 | | | 15.13 |
| 8 | 10 | 45 | 15.5 | 0.5 | 27 | | 2 | | | 13.19 |
| 9 | 10 | 45 | 17.5 | 0.5 | 27 | | | 0 | | 16.96 |
| 10 | 10 | 45 | 17 | 0.5 | 27 | | | 0.5 | | 16.41 |
| 11 | 10 | 45 | 16.5 | 0.5 | 27 | | | 1 | | 15.66 |
| 12 | 10 | 45 | 15.5 | 0.5 | 27 | | | 2 | | 14.36 |
| 13 | 10 | 45 | 17.5 | 0.5 | 27 | | | | 2.5 | 16.81 |
| 14 | 10 | 45 | 17 | 0.5 | 27 | 0.5 | | | 2.5 | 14.81 |
| 15 | 10 | 45 | 16.5 | 0.5 | 27 | | 1 | | 2.5 | 14.96 |
| 16 | 10 | 45 | 15.5 | 0.5 | 27 | | | 2 | 2.5 | 14.31 |

Referring to Table 2, referring to Experimental Examples II-1 to II-4, the greater silicon (Si) content, the lower thermal expansion coefficient of the metal alloy. When silicon was more added, silver was less used. For example, the alloy prepared according to Experimental Example II-1 may be used for low temperature calcination, the alloy prepared according to Experimental Example II-2 may be used for high temperature calcination. The alloys prepared according to Experimental Examples II-3 and II-4 may not be suitable for use as an alloy for fusing with porcelain due to their too low thermal expansion coefficients, when additional alloy elements are not used. Considering these results, the silicon content may be restricted to less than 1 weight%. However, the silicon content may be further increased by adding other alloy elements.

Referring to Experimental Examples II-5 to II-8, it is confirmed that the greater germanium (Ge) content, the smaller thermal expansion coefficient of the metal alloy. Compared to the case with silicon, when germanium was added, the thermal expansion coefficient was relatively less decreased. The alloys prepared according to Experimental Examples II-5 and II-6 may be used for low temperature calcination, and the alloy prepared according to Experimental Example II-7 may be used for high temperature calcination. The alloy prepared according to Experimental Example II-8 may not be suitable for use as an alloy for fusing with porcelain due to its too low a thermal expansion coefficient, when additional alloy elements are not used. Considering these results, the germanium content may be restricted to less than 2 weight%, for example, to a range of 1 weight% or lower. However, the germanium content may be further increased by adding other alloy elements.

Referring to Experimental Examples II-9 to II-12, it is confirmed that the greater zirconium (Zr) content, the smaller thermal expansion coefficient of the metal alloy. Compared to the case with germanium, when zirconium was added, the thermal expansion coefficient was relatively slightly less decreased. The alloys prepared according to Experimental Examples II-9 and II-11 may be used for low temperature calcination, and the alloy prepared according to Experimental Example II-12 may be used for high temperature calcination. Considering these results, it is confirmed that the zirconium content is about 2 weight% or slightly greater than 2 weight%. Furthermore, for high temperature calcinations, the zirconium content may be greater than 1 weight%. However, the zirconium content may be changed by adding other alloy elements.

Referring to Experimental Examples II-13 to II-16, when the cobalt (Co) is added, the thermal expansion coefficient of the metal alloys is maintained or slightly decreased. Accordingly, by adding silicon, germanium and/or zirconium and additionally, cobalt, the thermal expansion coefficient of the metal alloy may decrease and indium-caused high temperature calcinations deformation may be suppressed.

Table 3 shows composition (weight%) of the metal alloys prepared according to Experimental Example III.

**[Table 3]**

| Experimental Example III | Group 1 | Group 2 | Group 3 | Group 4 | Group 5 | | Group 6 | | | | | Group 7 | | | | | | | | | Group 8 |
|---|---|---|---|---|---|---|---|---|---|---|---|---|---|---|---|---|---|---|---|---|---|
| | Pd | Au | Ag | In | Zn | Ga | B | Ge | Zr | Si | Fe | Cr | Nb | Mg | Ti | Co | Al | Ta | Mo | Ir | Ru |
| 1 | bal. | 2 | 31 | 21.5 | | | 0.5 | | | | 0.1 | | | | | | | | | 0.1 | |
| 2 | bal. | 2 | 31 | 20.5 | | | | 1.4 | 0.1 | 0.1 | | | | | | | | | | | |
| 3 | bal. | 2 | 31 | 20.9 | | | | | 1.1 | | | | | | | | | | | | 0.1 |
| 4 | bal. | 2 | 31 | 20.5 | | | 1.2 | | 0.4 | | | | | | | | | | | | |
| 5 | bal. | 2 | 31 | 20.5 | | | 1 | | 0.5 | | | | | | | | | | | | 0.1 |
| 6 | bal. | 2 | 25 | 29.5 | 2.3 | | 1 | | | | 0.1 | | | | | | | | | 0.1 | |
| 7 | bal. | 2 | 26 | 29.1 | 2.5 | | 0.2 | | 0.5 | | | | | | | | | | | | |
| 8 | bal. | 5 | 21 | 23.4 | 2 | | | 0.1 | 3 | | | | | 0.5 | 3.9 | 0.1 | 1 | | | | |
| 9 | bal. | 5 | 18 | 24.8 | 2 | 3 | 1.8 | | 0.1 | | 0.1 | 3.4 | | | 1.5 | | | | 0.2 | 0.1 | |
| 10 | bal. | 10 | 20 | 26 | 2 | | | | 1.5 | | | | | | | | | 0.4 | | 0.1 | |
| 11 | bal. | 10 | 24 | 15.2 | 2 | 2 | 1.5 | | 0.7 | | | | | | 3.9 | 0.1 | 1.5 | | | 0.1 | |
| 12 | bal. | 5 | 30 | 17.3 | 2 | | | 1 | 1 | 0.1 | | 2.5 | | | | | 2 | | | 0.1 | |
| 13 | bal. | 3 | 29 | 19 | 2 | | 0.5 | | 1 | | | 2.0 | | 0.1 | | 0.5 | 0.8 | | | 0.1 | |
| 14 | bal. | 20 | 18 | 14 | | | 1.5 | | | 0.1 | | 0.5 | | | 4 | 0.8 | | | | 0.1 | |
| 15 | bal. | 20 | 18 | 13.8 | | 1 | 0.2 | | 0.8 | | 0.1 | 0.5 | | | 3.5 | | 1 | | | | 0.1 |
| 16 | bal. | 12 | 16 | 16.8 | 1 | 4 | | 1 | | | 0.2 | | | | 4.0 | 2.0 | 1.8 | | 0.1 | 0.1 | |
| 17 | bal. | 10 | 16 | 15 | 2 | 5 | 1.5 | | 1 | | | 3.9 | 0.1 | | | 0.4 | | | | 0.1 | |

Samples shown in Table 3 were prepared as follows: alloy components were dissolved and then injected into a graphite mold to form a mold product, and the mold product was extended by using a roller and then imprinted and cut to produce samples. The dissolving of alloy components may be performed with an appropriate reactor, for example, an induction chamber, and to prevent oxidation, the dissolving may be performed in vacuum conditions or while inert gas is injected. For example, a pressure inside the reactor may be controlled to be in a range of 10-4 to 10-3 torr, and in this state, argon or nitrogen gas may be injected thereinto.

The metal alloys for dental porcelain fusing prepared according to Experimental Examples III-1 to III-17 may be completely or partially constituted of Group 1 to Group VIII elements. In the above Experimental Examples, as the Group 1 element, platinum may be further added in addition to gold. In the above Experimental Examples, as the Group V element, in addition to the elements presented above, tin (Sn), selenium (Se), carbon (C), antimony (Sb), or copper (Cu) may be added as having similar functions as those of the elements presented above. As the Group VII element, the elements presented above, vanadium (V) or tungsten (W) may be added as having similar functions as those of the elements presented above. As the Group VIII element, the elements presented above, rhenium (Re), rhodium (Rh), lanthanum (La), osmium (Os), or bismuth (Bi) may be added as having similar functions as those of the elements presented above.

In the embodiments described above, the first adding element may be selected from Group VI elements, the second adding element may be selected from Group VII elements, the third adding element may be selected from Group V elements, and the fourth adding element may be selected from Group VIII elements.

Hereinafter, physical/mechanical properties, in vivo compatibility and an adhesive force of alloys prepared according to Experimental Examples shown in Table 2 are described in detail.

### Physical/mechanical properties evaluation _

Table 4 shows mechanical/physical properties of the alloys prepared according to Experimental Examples shown in Table 3. Such results were obtained by dissolving the alloys prepared according to Experimental Examples shown in Table 3 with an electric furnace used in technology laboratories or an oxygen-propane gas torch, and carrying out tests according to ISO9693 standard.

**[Table 4]**

| Experimental Example III | Physical characteristics | | |
|---|---|---|---|
| | Thermal expansion coefficient (10⁻⁶K⁻¹) | Melting point (°C) | Color |
| 1 | 17.2 | 1013 | yellow |
| 2 | 16.3 | 1021 | yellow |
| 3 | 16.5 | 1026 | yellow |
| 4 | 16.2 | 1030 | yellow |
| 5 | 15.9 | 1035 | yellow |
| 6 | 16.4 | 950 | yellow |
| 7 | 16.8 | 945 | yellow |
| 8 | 15.6 | 965 | yellow |
| 9 | 16.2 | 951 | yellow |
| 10 | 15.8 | 1037 | yellow |
| 11 | 14.7 | 1037 | yellow |
| 12 | 14.1 | 1033 | yellow |
| 13 | 15.2 | 1047 | yellow |
| 14 | 13.5 | 1129 | yellow |
| 15 | 14.2 | 1124 | yellow |
| 16 | 12.1 | 1140 | yellow |
| 17 | 13.1 | 1098 | yellow |

Referring to Tables 3 and 4, the alloys of Experimental Examples III-1 to III-17 were all yellow. Melting points of the alloys were in a range of 945 to 1047°C, and thermal expansion coefficient thereof were in a range of 12.1 to 17.2 10⁻⁶K⁻¹. Accordingly, it was confirmed that the alloys were suitable for low temperature or high temperature calcination. In Experimental Examples III-1 to III-17, palladium (Pd) was added in a content of 39 to 45 weight% for illustrative purpose. However, the Pd content may increase if contents of gold (Au) and silver (Ag) decrease.

In Experimental Examples III-1 to III-17, the gold (Au) content may be in a range of about 2 to 20 weight%, and the silver (Ag) content may be in a range of about 13 to 31 weight%. Within these content ranges of gold and silver, physical and mechanical properties of the alloys were all suitable for fusing with porcelain for teeth. However, the gold and silver contents may be outside the ranges as described above.

The melting point and the thermal expansion coefficient of the alloys may, first of all, significantly depend on the indium (In) content. Such effects of indium may be weakened or enhanced by other alloy elements. In the case of an alloy for fusing with porcelain for low temperature calcination, a low melting point is required although a thermal expansion coefficient is relatively high. On the other hand, in the case of an alloy for fusing with porcelain for high temperature calcination, a relatively low thermal expansion coefficient is required although a melting point is relatively high.

For example, a melting point of the alloy for low temperature calcination may be in a range of 920 to 1040°C, and a thermal expansion coefficient thereof may be in a range of 15.5 x 10⁻⁶ to 17.5 x 10⁻⁶ K⁻¹. From this point, the alloys of Experimental Examples III-1 to III-9 may be used for low temperature calcination. In this regard, the indium content may be in a range of about 20.3 to 30.0 weight%, and for example, 20.5 to 29.5 weight%.

In addition, when the melting point is restricted to 1000°C or lower, as in the case of Experimental Example III-6 to III-9, the indium content may be in a range of about 23.0 to 30.0 weight%. Furthermore, under strict conditions, for example, when a melting point is restricted to 1000°C or lower and a thermal expansion coefficient is restricted to a range of 16.0 x 10⁻⁶ to 17.2 x 10⁻⁶ K⁻¹, the indium content may be in a range of about 24 to 30 weight%.

Experimental Examples III-8 and III-9, compared to Experimental Examples III-1 to III-7, further include a Group VII element (second adding element), and accordingly, although the indium content is relatively low, a melting point is relatively low compared to those in Experimental Examples III-1 to III-7. From this point, it is confirmed that the Group VII element may contribute to a wider indium content range in an alloy for low temperature calcination and a lower melting point of the alloy.

An alloy for high temperature calcination may have a melting point of 1030 to 1200 °C, for example, 1030 to 1050 °C, and a thermal expansion coefficient of 12.0 x 10⁻⁶ to 16.0 x 10⁻⁶ K⁻¹, for example, 13.0 x 10⁻⁶ to 15.5 x 10⁻⁶ K⁻¹, and for example, 13.9 x 10⁻⁶ to 15.5 x 10⁻⁶ K⁻¹. From this point, the alloys of Experimental Examples III-10 to III-17 may be used for high temperature calcination, and in this case, the indium content may be in a range of about 12.0 to 26 weight%. Meanwhile, the indium content in the alloys for high temperature calcination may be increased by increasing contents of other adding elements, for example, gold, platinum, or the Group VII element (second adding element). Gold, silver, or platinum may contribute to an increase in the melting point of the alloy. The Group VII element may contribute to a decrease in the melting point of the alloy and a reduction in deformation of an alloy due to increased indium content.

The third adding element (Group V element) was added, as described above, in a content of 10 weight% or lower, for example, 7 weight% or lower in consideration of rigidity and corrosion resistance characteristics of an alloy.

The fourth adding element (Group VIII element) was added, as described above, in a content of 2 weight% or lower, for example, 0.5 weight% or lower in consideration of grain refinement of an alloy.

### In vivo compatibility evaluation

In vivo compatibility tests of samples prepared according to Experimental Examples III-1 to III-17 shown in Table 3 were performed according to ISO/TR 7455-1984 standard.

### <1> Acute toxicity test

The samples were prepared in powder, and suspended at various concentrations in water-soluble methyl cellulose, and then orally administered into Albino rat. For 2 weeks, toxicity effects of the samples were recorded every day. As a result, LD50 was 8 g/kg, which means high in vivo stability.

### <2> Agar layer method

Fibroblast L-929 cells of Albino rat were inoculated into Eagle MEM culture (10% FBS) at a cell concentration of 5105 per 1 mL cell, and then cultured at a temperature of 37°C for 24 hours. After the culturing, a liquid component of a culture plate was removed by suctioning, and about 12 mL of Eagle culture supplemented with 1.0% w/v agar was placed in the culture plate. 3 mL of 0.02% w/v neutral red phosphate buffer saline solution injection liquid was added to the agar plane, and then the cells were incubated with a 5% carbon dioxide apparatus at a temperature of 37°C for 2 to 3 hours. After the culturing, excess staining liquid was removed, and then, the result cells were incubated with a 5% carbon dioxide apparatus at a temperature of 37°C for 4 hours. After the culturing, 10100.1 mm-size thin films of the samples prepared according to Experimental Examples III-1 to III-5 were placed on 6 planes of the culture cells that sufficiently absorbed pigment. Of the planes, one plane was used as a negative control group. Thereafter, the planes were cultured with a 5% carbon dioxide incubator at a temperature of 37°C for 24 hours. The response of the culture cells was evaluated whether red-stained cells show a decrease in staining under and surrounding a subject when a culture dish was observed on the white background. Evaluation results show that cell death was similar to that of the negative control group. Accordingly, it was confirmed that the alloys have excellent in vivo compatibility.

### Evaluation on binding force with Porcelain

The binding tests between porcelain and the samples prepared according to Experimental Examples III-1 to III-17 shown in Table 3 were performed according to ISO 9693 standard. Test results show that all subjects retain 50% or more of porcelain in a central 1/3 thereof. Accordingly, it was confirmed that the samples had high binding force with porcelain.

Table 5 shows compositions (weight%) and physical characteristics of metal alloys of Experimental Example V.

**[Table 5]**

| Experimental Example IV | Group 1 | Group 2 | | Group 3 | Group 4 | Group 5 | | | Group 6 | | Group 7 | Physical Properties | | | | |
|---|---|---|---|---|---|---|---|---|---|---|---|---|---|---|---|---|
| | Pd | Au | Pt | Ag | In | Zn | Cu | Ga | Ge | Si | Co | Thermal Expansion Coefficient (10⁻⁶K⁻¹) | Melting point (°C) | Calcination range | Yellow level | Calcination deformation |
| 1 | 50 | 2 | | 23 | 21 | 2 | | | | | | 17.03 | 1030 | Low temperature | Low | X |
| 2 | 40 | 2 | | 25 | 30 | 3 | | | | | | 18.05 | 943 | X | Middle | X |
| 3 | 40 | 2 | | 24.6 | 30 | 3 | | | | 0.4 | | 16.20 | 945 | Low temperature | Middle | X |
| 4 | 40 | 2 | | 24 | 30 | 3 | | | 1 | | | 16.40 | 943 | Low temperature | Middle | X |
| 5 | 45 | 15 | | 16.5 | 21 | 2 | | | | 0.5 | | 14.4 | 1070 | High temperature | Middle | O |
| 6 | 43.8 | 10 | 0.8 | 15.7 | 24 | 0.7 | | | | 0.6 | 3.2 | 13.90 | 1180 | High temperature | Low | X |
| 7 | 38 | 10 | 1 | 16.6 | 28 | 0.5 | | | | 0.5 | 2.5 | 14.90 | 1100 | High temperature | Lower middle | X |
| 8 | 45 | 15 | | 14.5 | 21 | 2 | | | | 0.5 | 2 | 14.1 | 1180 | High temperature | Lower middle | X |
| 9 | 21.8 | 40 | 1 | 15.4 | 18 | 0.5 | 1 | 1 | | 0.6 | 1.5 | 14.14 | 11.5 | High temperature | High | X |
| 10 | 24.8 | 35 | 1 | 16.5 | 19 | 0.5 | 1 | 1 | | 0.6 | 1.5 | 14.22 | 1110 | High temperature | High | X |
| 11 | 24 | 30 | 1 | 20 | 20 | 0.6 | 1.6 | 1.6 | | 0.6 | 2 | 14.54 | 1096 | High temperature | Upper Middle | X |
| 12 | 35 | 17 | 0.5 | 15.5 | 27 | 0.5 | 1.6 | 1.6 | | 0.6 | 2.2 | 14.89 | 1082 | High temperature | Upper Middle | X |
| 13 | 33 | 15 | 1 | 21.9 | 24 | 0.5 | 2 | 2 | | 0.6 | 1.8 | 14.86 | 1101 | High temperature | Middle | X |
| 14 | 35 | 13 | 1 | 21 | 25 | 0.5 | 2 | 2 | | 0.6 | 1.8 | 14.87 | 1102 | High temperature | Middle | X |
| 15 | 38 | 10 | 1 | 16.6 | 28 | 0.5 | 2 | 2 | | 0.6 | 2.5 | 14.90 | 1100 | High temperature | Middle | X |
| 16 | 35 | 10 | 1 | 21 | 26 | 0.5 | 2.6 | 2.6 | | 0.6 | 3 | 14.89 | 1099 | High temperature | Middle | X |
| 17 | 38 | 6 | 1.4 | 19.2 | 28 | 0.5 | 3 | 3 | | 0.6 | 3 | 14.95 | 1100 | High temperature | Lower middle | X |
| 18 | 14 | 60 | 1 | 6.6 | 15 | 0.5 | 2 | | | 0.2 | 2 | 14.98 | 1095 | High temperature | High | X |
| 19 | 5.8 | 84 | 0.7 | 0.5 | 8.2 | 0.2 | 0.2 | | | 0.18 | 0.18 | 15.03 | 1073 | High temperature | High | X |

In the Experimental Examples described above, the first adding element may be selected from Group VI elements, the second adding element may be selected from Group VII elements, and the third adding element may be selected from Group V elements.

Referring to Table 5, it is confirmed that the alloy of Experimental Example V-1 in which the indium content is about 21% and the Group V element is included is suitable for low temperature calcination. However, Experimental Example V-1 has, compared to Experimental Example V-2, weak expression of yellow, and this is because the indium content is relatively low. However, as Experimental Example V-2, when only indium content increases, the thermal expansion rate substantially increases, leading to making low temperature calcination impossible.

From this point, as Experimental Examples V-3 to IV-19, the addition of the Group VI element may be effective for offsetting the increase in thermal expansion rate due to the addition of indium. From this point, it is considered that Experimental Examples V-3 and IV-4 are used for low temperature calcination and express a middle-level yellow.

Meanwhile, Experimental Examples V-5 to IV-19 in which the indium content is about 30 weight%, of Group VI elements (first adding element), the content of silicon is 0.5 weight% or more, are suitable for high temperature calcination. In consideration that about 80% of commercial ceramic porcelain is suitable for high temperature calcination, the metal alloys of these Experimental Examples used for high temperature calcination may be suitable for fusing with porcelain.

However, the alloy of Experimental Example V-5 that does not include cobalt, a Group VII element (second adding element) experienced calciantion deformation due to the flowing down of indium during fusing with porcelain. Accordingly, to perform high temperature calcination, a metal alloy needs to include an appropriate content of the Group VI element, and furthermore, to prevent calcination deformation during high temperature calcination, a metal alloy needs to include an appropriate content of the Group VII element. For example, the cobalt content may be controlled to be 5 weight% or lower.

However, due to the addition of the Group VII element, for example, cobalt, as Experimental Example V-6 to IV-8, the yellow expression level of the metal alloy was downgraded to lower middle or low. In consideration of this problem, as Experimental Examples V-9 to IV-16, the Group II element and the Group V element, in particular, copper (Cu) was added or a content thereof was increased, so as to upgrade the yellow expression level of the metal alloy to middle. However, the metal alloy of Experimental Example V-17 including a relatively low gold content showed a middle of the yellow expression level. For example, the copper content may be controlled to be 5 weight% or lower.

In particular, as Experimental Examples V-9, IV-10, IV-18 and IV-19, when the content of the Group II element, for example, gold was increased, copper was added as the Group V element, and the content of palladium was decreased, the metal alloy showed high level of yellow expression. Accordingly, it was confirmed that a meal alloy for high temperature calcination may have a gold content of about 85%. In this case, however, the increased gold content may lead to high costs. Accordingly, the gold content may be appropriately controlled in consideration of costs.

The dental metal alloys prepared according to the above Experimental Examples may be fused with ceramic porcelain for use in dental prosthetic appliances. Selectively, the dental metal alloys may have a stronger binding force with ceramic porcelain during binding by experiencing a pretreatment. Typically, when ceramic porcelain and a metal alloy are calcined, excess oxidation films may be interposed therebetween to prevent the binding between ceramic porcelain and a metal alloy. In particular, indium in the metal alloy may be oxidized during high temperature calcination.

From this point, by pretreating, prior to high temperature calcination, the metal alloy to remove a metal oxide on a surface of the metal alloy, formation of excess oxide between ceramic porcelain and the metal alloy is prevented to increase their chemical binding. In addition, by performing surface-etching to form pores in the surface of the metal alloy, a physical binding between the metal alloy and the ceramic porcelain is induced to increase a binding force. For example, the pretreatment may include wet-etching for the removal of the metal oxide. For example, wet-etching may be performed with an aqueous solution of a fluoric acid and a nitric acid. An etching time may be appropriately controlled to be a few to several minutes according to the metal oxide content in the metal alloy.

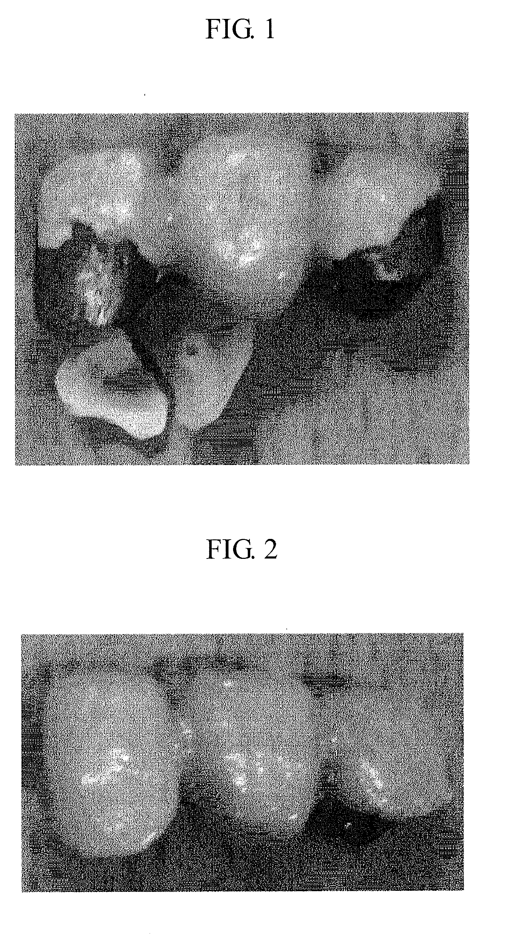

FIG. 1 shows a picture of a dental prosthetic appliance prepared by calcination without a pretreatment according to Comparative Example. FIG. 2 is a picture of a dental prosthetic appliance that is prepared by clacination with a pretreatment according to Experimental Example. The metal alloy prepared according to Experimental Example was molded in a tooth shape, and its calcination temperature with respect to ceramic porcelain may be in a range of about 900 to 980 °C.

As described in FIG. 1, when a pretreatment was not performed, the ceramic porcelain was substantially separated from the tooth-shaped metal alloy. However, as shown in FIG. 2, when a pretreatment was performed for an appropriate period of time, the ceramic porcelain was not separated from the tooth-shaped metal alloy or was not broken

It should be understood that the exemplary embodiments described therein should be considered in a descriptive sense only and not for purposes of limitation. Descriptions of features or aspects within each embodiment should typically be considered as available for other similar features or aspects in other embodiments.

## Claims

1. A metal alloy for dental porcelain fusing, the metal alloy comprising:
gold (Au) in a content that is greater than 0 and equal to or smaller than 35 weight%;
silver (Ag) in a content of 5 to 35 weight%;
indium (In) in a content of 6 to 40 weight%;
palladium (Pd) in a content of 30 to 70 weight%; and
at least one element selected from the group consisting of boron (B), germanium (Ge), zirconium (Zr), silicon (Si) and iron (Fe) in a total content that is greater than 0 and equal to or smaller than 5.0 weight%.

2. The metal alloy of claim 1, wherein the indium content is in a range of 12.0 to 35 weight%.

3. The metal alloy of claim 1, further comprising at least one element selected from the group consisting of zinc (Zn), tin (Sn), gallium (Ga), selenium (Se), carbon (C), antimony (Sb), and copper (Cu) in a total content that is greater than 0 and equal to or smaller than 10.0 weight%.

4. The metal alloy of claim 1, further comprising at least one element selected from the group consisting of iridium (Ir), rhenium (Re), rhodium (Rh), ruthenium (Ru), lanthanum (La), osmium (Os), and bismuth (Bi) in a total content of greater than 0 and equal to or smaller than 2 weight%.

5. The metal alloy of claim 1, further comprising at least one element selected from the group consisting of chromium (Cr), niobium (Nb), magnesium (Mg), titanium (Ti), cobalt (Co), aluminum (Al), vanadium (V), tantalum (Ta), molybdenum (Mo) and tungsten (W) in a total content of greater than 0 and equal to or smaller than 10.0 weight%.

6. The metal alloy of claim 1, wherein indium (In) has a content of 12.0 to 26.0 weight%, and the metal alloy has a thermal expansion coefficient of 13.0 X 10⁻⁶ to 16.0 X 10⁻⁶ K⁻¹.

7. The metal alloy of claim 1, wherein indium (In) has a content of 20.3 to 30.0 weight%, and the metal alloy has a thermal expansion coefficient of 15.5 X 10⁻⁶ to 17.5 X 10⁻⁶ K⁻¹.

8. The metal alloy of claim 1, wherein gold (Au) has a content of 2 to 20 weight%, and the content of silver (Ag) is in a range of 13 to 31 weight%.

9. A metal alloy for dental porcelain fusing, the metal alloy comprising:
gold (Au) in a content that is greater than 0 and equal to or smaller than 35 weight%;
silver (Ag) in a content of 5 to 35 weight%;
indium (In) in a content of 12.0 to 35.0 weight%;
at least one element selected from the group consisting of zinc (Zn), tin (Sn), gallium (Ga), selenium (Se), carbon (C), antimony (Sb), and copper (Cu) in a total content of greater than 0 and equal to or smaller than 10.0 weight%;
at least one element selected from the group consisting of boron (B), germanium (Ge), zirconium (Zr), silicon (Si) and iron (Fe) in a total content that is greater than 0 and equal to or smaller than 10.0 weight%;
at least one element selected from the group consisting of chromium (Cr), niobium (Nb), magnesium (Mg), titanium (Ti), cobalt (Co), aluminum (Al), vanadium (V), tantalum (Ta), molybdenum (Mo) and tungsten (W) in a total content of greater than 0 and equal to or smaller than 10.0 weight%;
at least one element selected from the group consisting of iridium (Ir), rhenium (Re), rhodium (Rh), ruthenium (Ru), lanthanum (La), osmium (Os), and bismuth (Bi) in a total content of greater than 0 and equal to or smaller than 2 weight%; and
residual palladium (Pd) and inevitable impurities.

10. The metal alloy of claim 9, wherein indium (In) has a content of 12.0 to 26.0 weight%, and the metal alloy has a melting point of 1030 to 1200°C.

11. The metal alloy of claim 9, wherein indium (In) has a content of 20.3 to 30.0 weight%, and the metal alloy has a melting point of 920 to 1040°C.

12. A metal alloy for dental porcelain fusing, the metal alloy comprising:
gold (Au) in a content that is greater than 0 and equal to or smaller than 85 weight%;
silver (Ag) in a content that is greater than 0 and equal to or smaller than 35 weight%;
indium (In) in a content of 6 to 40 weight%;
palladium (Pd) in a content of 5 to 70 weight%;
at least one first element selected from the group consisting of boron (B), germanium (Ge), zirconium (Zr), silicon (Si) and iron (Fe) in a total content that is greater than 0 and equal to or smaller than 5.0 weight%; and
at least one second element selected from the group consisting of chromium (Cr), niobium (Nb), magnesium (Mg), titanium (Ti), cobalt (Co), aluminum (Al), vanadium (V), tantalum (Ta), molybdenum (Mo) and tungsten (W) in a total content of greater than 0 and equal to or smaller than 10.0 weight%.

13. The metal alloy of claim 12, wherein the at least one element comprises silicon (Si) in a content that is greater than 0 and smaller than 1.0 weight%.

14. The metal alloy of claim 12, wherein the at least one second element comprises cobalt (Co) in a content of 0 to 5.0 weight%, and a calcination temperature of the metal alloy and ceramic porcelain is in a range of 900 to 980°C.

15. The metal alloy of claim 12, wherein the at least one element comprises silicon (Si) in a content that is greater than 0 and smaller than 1.0 weight%, and the at least one second element comprises cobalt (Co) in a content of 0 to 5.0 weight%.

16. The metal alloy of claim 13, further comprising at least one third element selected from the group consisting of zinc (Zn), tin (Sn), gallium (Ga), selenium (Se), carbon (C), antimony (Sb), and copper (Cu) in a total content that is greater than 0 and equal to or smaller than 10.0 weight%.

17. The metal alloy of claim 16, wherein the at least one element comprises silicon (Si) in a content that is greater than 0 and smaller than 1.0 weight%, the at least one second element comprises cobalt (Co) in a content of 0 to 5.0 weight%, and at least one third element comprises a copper (Cu) in a content that is greater than 0 and equal to or smaller than 5.0 weight%.

18. A dental prosthetic appliance manufactured by using the metal alloy of any one of claims 1-17.

19. A method of manufacturing a dental prosthetic appliance, the method comprising:
providing the metal alloy of any one of claims 1-17;
pretreating the metal alloy to remove a metal oxide on a surface thereof; and
fusing ceramic porcelain on the metal alloy at a calcination temperature range of 900 to 980°C.

20. The method of claim 19, wherein the metal oxide is removed by the heat treatment by wet etching an aqueous solution of a nitric acid and a fluoric acid.
